# EUROPÄISCHE PATENTSCHRIFT

(11) **EP 1 377 211 B1**
(45) Veröffentlichungstag und Bekanntmachung des Hinweises auf die Patenterteilung: **02.12.2009**
(21) Anmeldenummer: 02737788.6
(22) Anmeldetag: 09.04.2002
(51) Int. Cl.: A61B 5/05

(54) **VERFAHREN UND EINRICHTUNG ZUM ERFASSEN VON FUNKTIONS- UND STOFFWECHSELDATEN EINES LEBENDEN KÖRPERS**
METHOD AND DEVICE FOR DETECTING FUNCTIONAL AND METABOLIC DATA OF A LIVING ORGANISM
PROCEDE ET DISPOSITIF DE DETECTION DE DONNEES DE FONCTIONNEMENT ET DE METABOLISME D'UN ORGANISME VIVANT

(30) Priorität: 12.04.2001 DE 10119527
(43) Veröffentlichungstag der Anmeldung: 07.01.2004
(73) Patentinhaber: Texmed GmbH, 14482 Potsdam (DE)
(72) Erfinder: GEUTEBRÜCK, Ernst, 14772 Brandenburg (DE)
(74) Vertreter: Bittner, Thomas L.
(86) Internationale Anmeldenummer: PCT/DE2002/001378
(87) Internationale Veröffentlichungsnummer: WO 2002/082983

(56) Entgegenhaltungen:
- EP-A- 1 091 215
- WO-A-00/15110
- WO-A-95/20349
- WO-A-96/33651
- WO-A-98/28039
- WO-A-99/23945
- CH-A- 352 781
- DE-A- 1 616 006
- DE-A- 19 518 511
- FR-A- 2 750 029
- US-A- 6 047 203
- US-A- 6 088 615

## Beschreibung

Die Erfindung betrifft ein Verfahren zur mobilen oder stationären Erfassung von Körperfunktions- und Stoffwechseldaten eines lebenden Körpers sowie eine zweckmässig gestaltete Einrichtung zur Durchführung dieses Verfahrens.

Es ist eine Vielzahl von Einrichtungen bekannt, deren Messtechniken sich generell auf die indirekten Auswertungen von Patientenblut beziehen, welches entnommen werden muss. Die Handhabung und Auswertung ist schmerzhaft, umständlich und sollte mehrmals täglich mit besonderer Genauigkeit durchgeführt werden. Im Berufsleben sowie bei älteren Patienten bereitet diese Prozedur oft grosse-Schwierigkeiten. Aus diesem Grunde werden die Tests oft in zu geringer Frequenz oder falsch durchgeführt, sodass die Ergebnisse den beobachtenden Ärzten oft nur lückenhaft zur Verfügung stehen. Bekannt sind ebenfalls tragbare Messgeräte bekannt, insbesondere Messgeräte beispielsweise nach den Schriften DE 196 39 224 und DE 196 39 228 bekannt, wo die Bestimmung der Konzentration mindestens einer Substanz mit Hilfe einer Messoptik und einer Auswerteschaltung gelesen wird. Ferner ist ein Verfahren zur Bestimmung der Zuckerkonzentration nach der Schrift DE 32 28 551 bekannt, wo Zucker in Gegenwart störender Fremdsubstanzen mittels eines, eine Messelektrode mit vorgelagerter Membran aufweisenden elektrokatalytischen Zuckersensors festgestellt wird. Dabei ist die Messelektrode auf ein Reaktionspotential und auf ein Messpotential potentiostatiert und der während der Messperiode fliessende Strom wird als Messsignal ausgewertet. Die Erfindung sieht dazu vor, dass der Messelektrode nach dem Reaktionspotential und vor dem Messpotential kurzzeitig ein drittes Potential aufgeprägt wird, das negativer ist als das Messpotential.

Es ist auch eine transcutane, unblutige Konzentrationsbestimmung von Substanzen im Blut nach der DE-Schrift 195 18 511 bekannt, wo im Blut eines Patienten zu bestimmende Substanzen, wie Lactat, Glucose, Cholesterin, Blutzucker, Alkohol, Drogen oder dergleichen durch
a) ein der Menge einer Substanz und der Wassermenge einer gegebenen Körperregion entsprechendes Signal, das mittels spektroskopischer Methoden erzeugt wird, gemessen wird,
b) die Konzentration im Wasser durch Verhältnisbildung des Signalwertes der Substanz- und Wassermenge ermittelt wird, und
c) hieraus der Blutkonzentrationswert errechnet wird.

Durch die Schrift DE 195 19 05 1 sind ferner ebenfalls Verfahren und Vorrichtung zur polarimetrischen Bestimmung von Stoffen im menschlichen Körper bekannt, wo aus einem mit linear polarisiertem Licht bestrahlten durchbluteten Bereich des Körpers austretendes Streulicht analysiert und aus der Korrelation zwischen dem so ermittelten Drehwinkel der Polarisation und der Blutzuckerkonzentration der aktuelle Blutzuckerwert bestimmt wird.

Das Dokument US 6,088,615 A beschreibt eine Impedanzmessung zwischen dem linken und dem rechten Arm sowie dem linken und dem rechten Fuß eines lebenden Körpers. Hierbei wird der lebende Körper aktiv mit Hochfrequenzsignalen über Elektroden beaufschlagt, wodurch durch den Körper ein elektrischer Strom fließt.

Aus dem Dokument WO 00/15110 A geht ein Verfahren und eine Vorrichtung zum Erfassen biometrischer Daten zur Personenidentifizierung hervor. Die Messung wird ausgeführt, indem ein lebender Körper mit Signalen beaufschlagt wird, die zu einem Stromfluss führen, welcher seinerseits mit Hilfe von Elektroden erfasst wird.

Das Dokument WO 99/23945 A beschreibt eine Vorrichtung zum Erfassen einer Impedanz eines Patienten, wobei eine Referenzelektrode und eine Messsonde auf die Hautoberfläche des Patienten aufgebracht sind. Über die Referenzelektrode oder die Messsonde wird dem Patienten dann ein elektrischer Strom appliziert, so dass der Strom durch die Haut fließt. Die daraus resultierende Potentialdifferenz zwischen der Elektrode und der Messsonde wird gemessen und der elektrische Widerstand der Haut bestimmt.

Aus dem Dokument DE 1 616 006 geht eine Schaltung zum Überwachen der Durchlässigkeit des Zellgewebes eines lebenden Körpers für eine körpereigene Flüssigkeit hervor.

Das Dokument CH 352781 A beschreibt ein Verfahren und eine Vorrichtung zur Feststellung von Eigenschaften und Verschiedenheiten von Körpern. Hierbei wird mittels über die Körperoberfläche bewegbaren Abtastelektroden das beim Anlegen einer Wechselspannung an den Körper entstehende elektrische Feld abgetastet.

Genereller Nachteil der Mehrzahl aller bekannten Lösungen ist somit die indirekte Auswertung von Blut, welches vom Patienten entnommen werden muss. Gleichfalls setzt die Handhabung der herkömmlichen invasiven Messmethodik ausserdem ein exaktes Hantieren während eines exakt definierten Zeitraumes voraus, was von bestimmten behinderten Menschen oder älteren Menschen nicht erfüllbar ist. Ein weiterer gravierender Nachteil aller herkömmlich bekannten und eingesetzten Geräte liegt in der ungenügenden Datenerfassung und (manuellen) Archivierung auf Patientenseite. Abgesehen davon, dass es neben einer mangelhaften Dateneintragung oft zu einer "Schönung" der Resultate kommt, ist auch das "Papier" als Dokumentation für die detaillierte statistische Auswertung durch den notwendigen zeitlichen Aufwand und fehlender Software für den Mediziner ungeeignet.

Die Erfindung hat sich daher zur Aufgabe gestellt, ein Verfahren und eine Einrichtung zu entwickeln, welche an einem lebenden Körper eine schmerzfreie, einfach zu handhabende, beliebig oft durchführbare Selbstkontrolle von Körperwerten bei gleichzeitiger individueller Datenerfassung ermöglicht.

Die Aufgabe wird gelöst durch ein Verfahren nach Anspruch 1 sowie einer Einrichtung nach Anspruch 25.

Auf der Hautoberfläche oder in deren Nähe oder in die Hautoberfläche des Probanden implantiert, sind alternierend als Sensor und als Aktor betriebene Elektroden aufgebracht. Diese registrieren ausgelöste und reflektierte Signale und zeichnen diese auf. Diese gespeicherten Signale werden einer nachgeschaltenen Datenverarbeitungseinrichtung aufgegeben. Aus den ermittelten Werten werden über den zeitlichen Verlauf Summenwerte gebildet. Diese Summenwerte werden Referenzwerten zugeordnet und mit diesen zusammen regelmässig angezeigt, damit ein Vergleich möglich ist. Die Anzeige kann auf einem Bildschirm erfolgen, die Anzeige kann jedoch ebenso über eine Schreibeinrichtung erfolgen, wobei beide Einrichtungen mit einem Drucker zur Ausgabe der ermittelten Werte verbunden sein können. Ebenso können die ermittelten und abgeglichenen Werte einem Datenspeichersystem aufgegeben werden, damit sie vergleichenderweise zur weiteren Verfügung verbleiben und bei Bedarf abgerufen werden können.

Die Signale zur Beurteilung des Probanden können kontinuierlich oder diskontinuierlich gewonnen werden.

Die vorhandenen natürlichen und künstlichen elektrischen, elektrochemischen und elektromagnetischen Felder sind physikalische Parameter. Als relevanter physikalischer Parameter kann die Feldstärke dienen. Es ist ebenso möglich, die Dichte von Feldlinien zu registrieren, eine Potentialdifferenz, eine Stromstärke, eine elektrische Spannung oder wenigstens zwei Grössen in der Kombination. Insbesondere sind für Zwecke der Speicherung und Dokumentation Körpertemperatur, der Hautwiderstand sowie der Gasaustausch durch die Haut in der Zeiteinheit relevant.

Unter Hinweis auf spezifische relevante Körperpartien des Probanden kann die Erfassung punktförmig, linien-oder flächenförmig vorgenommen werden. Des weiteren kann die Anordnung den relevanten Hautpartien angepasst sein, beispielsweise in Form einer Matrix. Ebenso können mindestens die Elektroden in den Körper des Probanden implantiert sein. Nicht eingegangen ist auf eine wie auch immer ausserhalb des Körpers des Probanden befindliche Anordnung der Elektroden.

Eine erfindungsgemässe Einrichtung zur Durchführung des erfindungsgemässen Verfahrens besteht zunächst aus als Sensoren und Aktoren betreibbaren Elektroden, die in Bezugnahme auf die Hautoberfläche des Probanden angeordnet sind. Diese sind elektrisch/elektronisch mit einer Operationsverstärkerstufe vorgelagerten analogen Umschalteinrichtung verbunden. Die Operationsverstärkerstufe besteht aus einem Impedanzwandler-Vorverstärker mit Filter. Der der Konditionierung dienenden Operationsverstärkerstufe ist ein Analog-Digital-Wandler mit digitalem Signalprozessor nachgeschalten. Dieser ist wiederum mit einer die normalisierten und digitalisierten Messwerte weiterverarbeitenden und speichernden Rechnerstufe zusammengeschalten, weshalb die Rechnerstufe vorzugsweise ebenso ein integriertes Speichermodul aufweisen kann.

Sind die einzelnen Baueinheiten der erfindungsgemässen Einrichtung räumlich getrennt angeordnet, kann die Einrichtung die Daten zwischen diesen Baueinheiten drahtlos übertragend ausgebildet sein. Adäquat kann dazu ebenso eine die Daten übertragende optische Kommunikationsschhittstelle ausgebildet sein.

Die Operationsverstärkerstufe ist den physikalischen Eigenschaften der Elecktroden angepasst. Ebenso sieht eine erfindungsgemässe Vorzugsvariante vor, dass der Analog-Digital-Wandler mit digitalem Signalprozessor entsprechend der Anzahl der eingesetzten Primärkontakte skalierbar ausgebildet ist. Vorzugsweise kann die Rechnerstufe ebenso skalierbar ausgeführt sein.

Eine weitere erfindungsgemässe Vorzugsvariante sieht vor, dass die Rechnerstufe den der adaptiven Stimulierung dienenden Generator und damit die Umschaltung zwischen Sensor- und Aktorbetrieb steuernd ausgebildet ist.

Vorzugsweise ist der Rechnerstufe ein Display nachgeordnet, damit die erhaltenen Schaubilder einer Interpretation unterzogen werden können. Ebenso kann der Rechnerstufe ein Depot-Regulator diese steuernd nachgeordnet sein.

Um Störsignale zu eliminieren, besitzt die Operationsverstärkerstufe ein adaptives Filterelement, damit erkannte Störsignale eliminiert werden können.

Wenigstens einzelne Baueinheiten können in den lebenden Körper implantiert sein. Bei hinreichender Miniaturisierung kann ebenso die gesamte Einheit im lebenden Körper befindlich angeordnet sein.

Ausgangspunkt zur Entwicklung des erfindungsgemässen Verfahrens sowie dem Einsatz der erfindungsgemässen Einrichtung war, dass ein natürliches Humanfeld vorhanden ist, das im lebenden Körper nachweisbar ist. Es wurde ebenso festgestellt, dass der korrelierbare Einfluss bestimmter Stoffe im Blut als Anomalie im natürlichen Humanfeld messbar ist. Damit ist eine vollständig nichtinvasive Anwendung des erfindungsgemässen Verfahrens sowie der erfindungsgemässen Einrichtung vorteilhaft gegeben.

Damit ist es möglich, über ein nicht-invasives Verfahren die laufende Kontrolle der Körperwerte eines lebenden Körpers wesentlich zu vereinfachen. Ebenso ist eine kontinuierlich stabile Einstellung von Körperwerten während einer Behandlung_gegeben.

Ebenso ist es möglich, von einem scheinbar gesunden Lebewesen Körperwerte zur Beurteilung von Stoffwechselerscheinungen, wie Veränderungen von Blutsubstanzen, Blutalkohol, Glucose, Muskeleffizienz, Hautwiderstand, Hautdurchblutung, Schmerzlokation und dergleichen Werte zu gewinnen.

Sämtliche Daten können personenbezogen gespeichert, analysiert, visualisiert und als individuelle Tages-, Wochen- und/oder beliebige Zeitprofile von physikalischen Daten und deren Auswirkungen, wie z. B. Konzentration von Substanzen und dergleichen, dargestellt und auch in andere Datensysteme fernübertragen werden.

Eine üblichen Methoden anhaftende Aufwendigkeit durch hohen Papierauf wand bei der Sammlung ermittelter Daten sowie damit verbundener Fehler wird durch die rechnergestützte Datenerfassung und einen ebenso möglichen Datentransfer zum weiterbehandelnden Spezialisten oder dergleichen beseitigt.

Die Erfindung soll im folgenden an einem Ausführungsbeispiel, in dem sowohl das Verfahren als auch die Einrichtung beispielhaft näher erläutert sind, dargestellt werden.

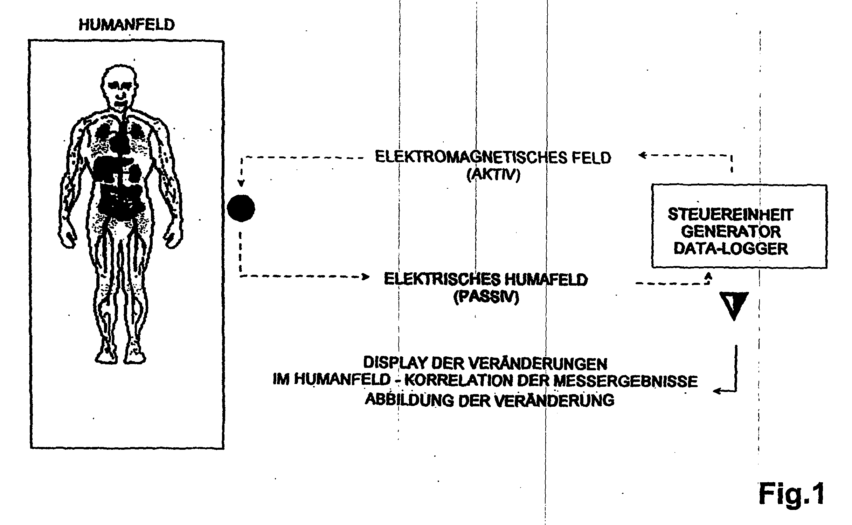

In den zugehörigen Zeichnungen zeigen
- Fig. 1: das Grundprinzip des Messablaufes bei Anwendung des erfindungsgemässen Verfahrens,

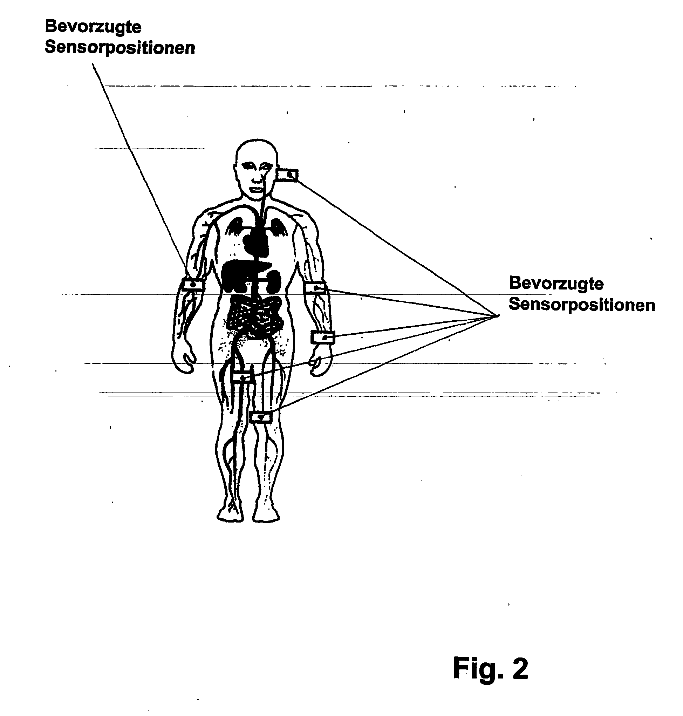

- Fig. 2: Darstellung bevorzugter Sensorpositionen an einem menschlichen Körper,

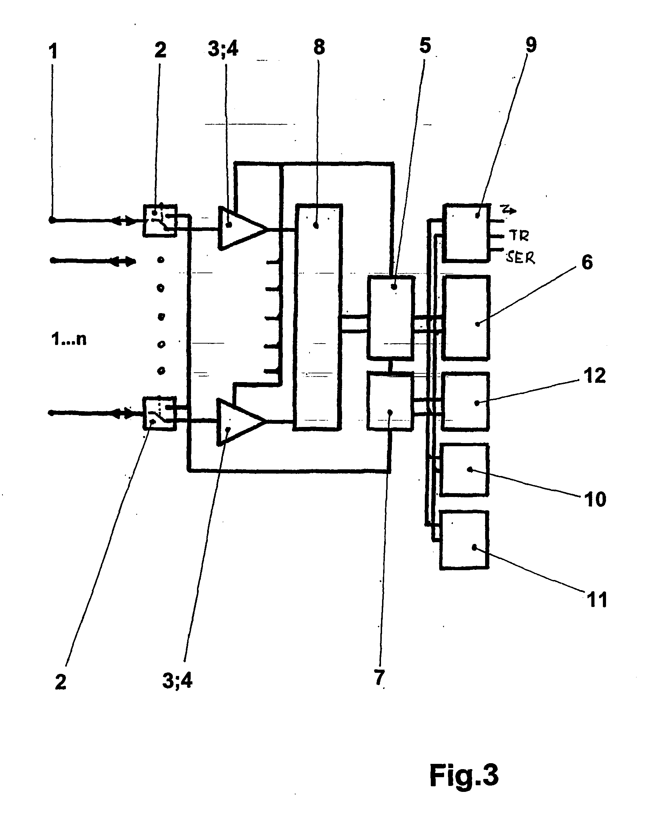

- Fig. 3: Prinzipschaltschema einer erfindungsgemäss aufgebauten Einrichtung.

Auf der Körperoberfläche eines Probanden sind aus leitfähigem Material eine Zahl von 1...n Elektroden 1 aufgebracht. Als leitfähiges Material können alle körperverträglichen edlen Metalle (Gold, Silber, Platin) oder aber leitfähige Kunststoffe oder Keramiken zum Einsatz kommen. Als Trägermaterial für die Elektroden können alle isolierenden Stoffe wie Kunststoffe oder Textilien dienen. - Beispielhaft sind das 6 Elektroden, die miniaturisiert in leitfähigen Gummi eingebettet sind, der wiederum zu seiner Halterung von einem dehnungsfähigen Polymer umfasst ist und sich so dem Körper des Probanden anpasst.

Auf dem Trägermaterial ist gleichzeitig die Elektronik mit angeordnet. Ebenso dient es zur Befestigung am Körper des Probanden.

Die Elektroden 1 sind sowohl als Sensor als ebenfalls als Aktor wirkend ausgeführt.

Die nachfolgende Baueinheit ist die Signalkonditionierung (A). Hier ist für jede Elektrode 1 eine Anordnung aus Ein-Kanal-Analogschalter 2, Impedanzwandler-Vorver-stärker 3 und Filter 4 vorhanden Letztere bilden zusammen als Einheit die Operationsverstärkerstufe.

Werden die Elektroden 1 als Sensor betrieben, wird das Sensorsignal dem Impedanzwandler-Vorverstärker 3 aufgegeben. Das nachgeschaltene Filter 4 erfüllt zwei Aufgaben - es soll zunächst die Störung durch induzierte Umweltfelder unterdrücken, des weiteren das zur Datenwandlung weiterzuleitende Signal begrenzen. Das Filter 4 ist beispielhaft mehrstufig aktiv konzipiert ausgeführt. Die Filterparameter werden durch einen digitalen Signalprozessor 5 und einen Prozessor 6 angepasst und nachgeführt. Damit steht ein zur Weiterverarbeitung geeignetes analoges Signal zur Verfügung.

Werden die Elektroden 1 als Aktor betrieben, sind diese über den Ein-Kanal-Analogschalter 2 mit dem Generator 7 gekoppelt.

In der Baueinheit C erfolgt die Datenwandlung. Hier ist in die Datenwandlungsstufe 8, die mit dem Signalprozessor 5 gekoppelt ist, ein skalierbares Interface integriert, welches den Betrieb von mehreren Elektroden 1 ermöglicht. Somit können die Elektroden 1 als Einzelelement oder als Matrix an der Datenwandlungsstufe 8 betrieben werden. Die Ansteuerung erfolgt durch den Prozessor 6.

Dem skalierbaren Interface ist ein hochauflösender schneller Analog-Digital-Wandler 8 nachgeschalten, der über ein Signalprozessorinterface verfügt. Der Analog-Digital- Wandler 8 wird direkt vom Signalprozessor 5 angesteuert und dient dem Signalprozessor 5 als Datenquelle.

Im Signalprozessor 5 wird der eingehende Datenstrom vorverarbeitet, indem Störsignale ermittelt und entfernt werden. Ebenso werden die Parameter für das adaptive Filter bestimmt.

Alle Ausgangsdaten werden der folgenden Prozessorstufe über ein Bussystem übergeben. Über einen Controlbus steuert der Prozessor 6 den Signalprozessor 5.

Der in dieser Baueinheit enthaltene Generator 7 wird ebenfalls über dieses Bussystem (Daten und Controlbus) gesteuert. Es können mehrere gespeicherte Signalverläufe aktiviert werden. Intensität und Frequenz sind weitere Parameter für den Generator 7. Werden neue Signalverläufe sythetisiert, so können diese benutzerspezifisch gespeichert werden.

Die Baueinheit D ist das sogenannte Controllersegment. Der Controller dient als zentrale Steuerinstanz im System. Er überwacht alle Systemfunktionen, generiert zyklisch Selbsttests und führt die Onlinekalibrierung des Gesamtsystems durch. Ebenso kann er Vergleichsmessungen mit der klassischen Analysetechnik überwachen.

Über das Bussystem sind die Komponenten Kommunikationsmodul 9, die Speichereinheit 12, das Systemdisplay 10 und ein Keyboard 11 mit dem Controller verbunden. Das Kommunikationsmodul 9 ist modular aufgebaut und somit an unterschiedliche Übertragungskanäle anpassbar. Ebenso ist es möglich, Soft- oder Hardwareprotokolle zu implementieren.

Die Speichereinheit 12 dient der langfristigen Speicherung der gewonnenen Daten und der temporären Zwischenspeicherung der aktuellen Daten. Die Speichereinheit 12 ist vorzugsweise in einen fest im System implementierten Speicher und einen erweiterbaren externen Speicher unterteilt.

Ein weiteres externes Speichermedium kann mit austauschbaren Speichermedien bestückt sein.

Ein spezifisches Auswerteprogramm übernimmt die personenbezogene Analyse der Messdaten und Programmschritte wie z. B. eine Korrelation mit z. B. personenbezogenen Referenzwerten, historischen Werten und dergleichen Abweichungen von Zeitwerten und dergleichen. Des weiteren führt das Auswerteprogamm Berechnungen durch, beispielsweise zur Ermittlung der Konzentration bestimmter chemischer Substanzen.

### Bezugszeichen:

| | |
|---|---|
| Elektroden | 1 |
| Ein-Kanal-Analogschalter | 2 |
| Impedanzwandler-Vorverstärker | 3 |
| Filter | 4 |
| Signalprozessor | 5 |
| Prozessor | 6 |
| Generator | 7 |
| Analog-Digital-Wandler | 8 |
| Kommunikationsmodul | 9 |
| Systemdisplay | 10 |
| Keyboard | 11 |
| Speichereinheit | 12 |

## Patentansprüche

1. Verfahren zur mobilen oder stationären Erfassung von Körperfunktions- und Stoffwechseldaten eines lebenden Körpers, wobei sich in dem lebenden Körper eine natürliche und künstliche elektrische, elektrochemische und elektromagnetische Felder beeinflussende Substanz befindet und wobei das Verfahren die folgenden Schritte umfasst:
- Erfassen von elektrischen Signalen, welche einer Änderung der natürlichen und künstlichen elektrischen, elektrochemischen und elektromagnetischen Feldern entsprechen, durch mittels einer vorgelagerten analogen Umschalteinrichtung (2) alternierend als Sensor und als Aktor betriebenen Elektroden, welche in Beziehung zu einer Hautoberfläche des lebenden Körpers gebracht werden,
- Registrieren und Aufzeichnen der erfassten Signale in einem Speicher und
- Übertragen der gespeicherten Signale an eine Datenverarbeitungseinrichtung.

2. Verfahren nach Anspruch 1, **dadurch gekennzeichnet, dass** das Erfassen der elektrischen Signale kontinuierlich erfolg.

3. Verfahren nach Anspruch 1 oder 2, **dadurch gekennzeichnet, dass** die Einwirkung der als Sensor und als Aktor betreibbaren Elektroden global erfolgt.

4. Verfahren nach einem der vorangehenden Ansprüche, **dadurch gekennzeichnet, dass** die elektrischen Signale natürlichen und künstlichen elektrischen, elektrochemischen und elektromagnetischen Feldern entsprechen, die mittels der Elektroden in punktförmiges Anordnung gemessen wurden.

5. Verfahren nach einem der Ansprüche 1 bis 3, **dadurch gekennzeichnet, dass** die elektrische Signale natürlichen und künstlichen elektrischen, elektrochemischen und elektromagnetischen Feldern entsprechen, die mittels der Elektroden in linienförmiger Anordnung gemessen wurden.

6. Verfahren nach einem der Ansprüche 1 bis 3, **dadurch gekennzeichnet, dass** die elektrischen Signale natürlichen und künstlichen elektrischen, elektrochemischen und elektromagnetischen Feldern entsprechen, die mittels der Elektroden in flächenformiger Anordnung gemessen wurden.

7. Verfahren nach einem der vorangehenden Ansprüche, **dadurch gekennzeichnet, dass** die elektrischen Signale einer Feldstärke entsprechen.

8. Verfahren nach einem der vorangehenden Ansprüche, **dadurch gekennzeichnet, dass** die elektrischen Signale einer Dichte von Feldlinien entsprechen.

9. Verfahren nach einem der vorangehenden Ansprüche, **dadurch gekennzeichnet, dass** die elektrischen Signale einer Potentialdifferenz entsprechen.

10. Verfahren nach einem der vorangehenden Ansprüche, **dadurch gekennzeichnet, dass** die elektrischen Signale einer Stromstärke entsprechen.

11. Verfahren nach einem der vorangehenden Ansprüche, **dadurch gekennzeichnet, dass** die elektrischen Signale einer elektrischen Spannung entsprechen.

12. Verfahren nach einem der vorangehenden Ansprüche, **dadurch gekennzeichnet, dass** die elektrischen Signale zur graphischen Darstellung bereitgestellt werden.

13. Verfahren nach einem der vorangehenden Ansprüche, **dadurch gekennzeichnet, dass** eine Körpertemperatur gespeichert wird.

14. Verfahren nach einem der vorangehenden Ansprüche, **dadurch gekennzeichnet, dass** ein Hautwiderstand gespeichert wird.

15. Verfahren nach einem der vorangehenden Ansprüche, **dadurch gekennzeichnet, dass** ein Gasaustausch durch die Haut je Zeiteinheit gespeichert wird.

16. Verfahren nach einem der vorangehenden Ansprüche, **dadurch gekennzeichnet, dass** die natürliche und künstliche elektrische, elektrochemische und elektromagnetische Felder beeinflussende Substanz Lactat ist.

17. Verfahren nach einem der Ansprüche 1 bis 15, **dadurch gekennzeichnet, dass** die natürliche und künstliche elektrische, elektrochemische und elektromagnetische Felder beeinflussende Substanz ein Alkohol ist.

18. Verfahren nach einem der Ansprüche 1 bis 15, **dadurch gekennzeichnet, dass** die natürliche und künstliche elektrische, elektrochemische und elektromagnetische Felder beeinflussende Substanz Cholesterin ist.

19. Verfahren nach einem der Ansprüche 1 bis 15, **dadurch gekenntzeichnet,** dass die natürliche und künstliche elektrische, elektrochemische und elektromagnetische Felder beeinflussende Substanz ein Stearin ist.

20. Verfahren nach einem der Ansprüche 1 bis 15, **dadurch gekennzeichnet, dass** die natürliche und künstliche elektrische, elektrochemische und elektromagnetische Felder beeinflussende Substanz ein Eiweiß ist.

21. Verfahren nach einem der Ansprüche 1 bis 15, **dadurch gekennzeichet,** dass die natürliche und künstliche elektrische, elektrochemische und elektromagnetische Felder beeinflussende Substanz Drogen sind.

22. Verfahren nach einem der Ansprüche 1 bis 15, **dadurch gekenntzeichnet,** dass die natürliche und künstliche elektrische, elektrochemische und elektromagnetische Felder beeinflussende Substanz Blutfette sind.

23. Verfahren nach einem der Ansprüche 1 bis 15, **dadurch gekennzeichnet, dass** die natürliche und künstliche elektrische, elektrochemische und elektromagnetische Felder beeinflussende Substanz Glukose ist

24. Verfahren nach einem der Ansprüche 1 bis 15, **dadurch gekennzeichnet, dass** die natürliche und künstliche elektrische, elektrochemische und elektromagnetische Felder beeinflussende Substanz Blutzucker ist.

25. Einrichtung zur Durchführung des Verfahrens nach einem der Ansprüche 1 bis 24, wobei:
- in Bezugnahme auf einer Hautoberfläche eines lebenden Körpers als Sensor und als Aktor betreibbare Elektroden (1) angeordnet sind,
- die Elektroden (1) elektrisch / elektronisch mit einer analogen Umschalteinrichtung (2) verbunden sind, welche einer Ope.rationsverstärkerstufe (3; 4) vorgelagert ist,
- einem Impedanz-Vorverstärker (3), welcher der Konditionierung dient, ein Analog-Digital-Wandler (8) mit digitalem Signalprozessor (5) nachgeschaltet ist und
- dieser mit einer die normalisierten und digitalisierten Messwerte weiterverarbcitenden und speichernden Rechnerstufe zusammengeschaltet ist.

26. Einrichtung nach Anspruch 25, **dadurch gekennzeichnet, dass** einzelne Baueinheiten räumlich getrennt angeordnet sind.

27. Einrichtung nach einem der Ansprüche 25 oder Anspruch 26, **dadurch gekennzeichnet, dass** die Baueinheiten die Daten drahtlos übertragend ausgebildet sind.

28. Einrichtung nach einem der Ansprüche 25 bis 27, **dadurch gekennzeichnet, dass** Baueinheiten die Daten über eine optische Kommunikationsschnittstelle übertragend ausgebildet sind.

29. Einrichtung nach einem der Ansprüche 25 bis 28, **dadurch gekennzeichnet, dass** die als Sensor und als Aktor betreibbaren Elektroden (1) in Gruppen angeordnet sind.

30. Einrichtung nach Anspruch 29, **dadurch gekennzeichnet, dass** die Elektroden (1) in Gruppen in einer Matrix angeordnet ausgebildet sind.

31. Einrichtung nach einem der Ansprüche 25 bis 30, **dadurch gekennzeichnet, dass** mindestens einzelne Baueinheiten des Systems in den lebenden Körper implantiert sind.

32. Einrichtung nach einem der Ansprüche 25 bis 31, **dadurch gekennzeichnet, dass** die Qperationsverstärkerstufe (3; 4) den physikalischen Eigenschaften der Elektroden angepasst ist.

33. Einrichtung nach einem der Ansprüche 25 bis 32, **dadurch gekennzeichnet, dass** der Analog-Digital-Wandler (8) mit digitalem Signalprozessor (5) nach der Anzahl der eingesetzten Primärkontakte skalierbar ausgebildet ist.

34. Einrichtung nach einem der Ansprüche 25 bis 33, **dadurch gekennzeichnet, dass** die Rechnerstufe skalierbar ausgeführt ist.

35. Einrichtung nach einem der Ansprüche 25 bis 34, **dadurch gekenntzeichnet,** dass die Rechnerstufe den der adaptiven Stimulierung dienenden Generator (7) und damit die Umschaltung zwischen Sensor- und Aktorbetrieb steuernd ausgebildet ist.

36. Einrichtung nach einem der Ansprüche 25 bis 35, **dadurch gekennzeichnet, dass** der Rechnerstufe ein Display (10) nachgeordnet ist.

37. Einrichtung nach einem der Ansprüche 25 bis 36, **dadurch gekennzeichnet, dass** die Rechnerstufe eine in sie integrierte Speichereinheit (12) aufweist.

38. Einrichtung nach einem der Ansprüche 25 bis 37, **dadurch gekennzeichnet, dass** die Operationsverstärkerstufe (3; 4) ein adaptives Filterelement (4) zur Eliminierung erkannter Störsignale aufweist.

39. Einrichtung nach Anspruch 38, **dadurch gekennzeichnet, dass** das adaptive Filterelement (4) mehrstufig aktiv konzipierend ausgeführt ist.

40. Einrichtung nach einem der Ansprüche 25 bis 39, **dadurch gekennzeichnet, dass** die Rechnerstufe mit einem Depot-Regulator steuernd verbunden ist.

41. Einrichtung nach Anspruch 40, **dadurch gekennzeichnet, dass** der Depot-Regulator innerhalb des lebenden Körpers angeordnet ist.

42. Einrichtung nach Anspruch 40, **dadurch gekenntzeichnet,** dass der Depot-Regulator außerhalb des lebenden Körpers angeordnet ist.

## Claims

1. Method for mobile or in-patient detection of bodily function and metabolic data of a living organism, wherein a substance is located in the living organism which influences natural and artificial electric, electrochemical and electromagnetic fields and wherein the method comprises the following steps:
- detecting electric signals that correspond to a change of the natural and artificial electric, electrochemical, and electromagnetic fields by electrodes which are alternately operated as sensor and as actuator by means of a upstream analog switching device (2) and which are related to a skin surface of the living organism,
- registering and storing the detected signals in a storage, and
- transmitting the stored signals to a data processing device.

2. Method according to claim 1 **characterized in that** the detection of the electric signals is carried out continuously.

3. Method according to claim 1 or 2 **characterized in that** the effect of the electrodes operable as sensor and as actuator occurs globally.

4. Method according to one of the preceding claims **characterized in that** the electric signals correspond to natural and artificial electric, electrochemical, and electromagnetic fields that are measured by the electrodes in a punctual arrangement.

5. Method according to one of the claims 1 to 3 **characterized in that** the electric signals correspond to natural and artificial electric, electrochemical, and electromagnetic fields that are measured by the electrodes in a linear arrangement.

6. Method according to one of the claims 1 to 3 **characterized in that** the electric signals correspond to natural and artificial electric, electrochemical, and electromagnetic fields that are measured by the electrodes in a laminar arrangement.

7. Method according to one of the preceding claims **characterized in that** the electric signals correspond to a field strength.

8. Method according to one of the preceding claims **characterized in that** the electric signals correspond to a density of field lines.

9. Method according to one of the preceding claims **characterized in that** the electric signals correspond to a potential difference.

10. Method according to one of the preceding claims **characterized in that** the electric signals correspond to an amperage.

11. Method according to one of the preceding claims **characterized in that** the electric signals correspond to an electric voltage.

12. Method according to one of the preceding claims **characterized in that** the electric signals are provided for a graphical representation.

13. Method according to one of the preceding claims **characterized in that** a body temperature is stored.

14. Method according to one of the preceding claims **characterized in that** a skin resistance is stored.

15. Method according to one of the preceding claims **characterized in that** a gas exchange through the skin per time unit is stored.

16. Method according to one of the preceding claims **characterized in that** the substance which influences natural and artificial electric, electrochemical, and electromagnetic fields is a lactate.

17. Method according to one of the claims 1 to 15 **characterized in that** the substance which influences natural and artificial electric, electrochemical, and electromagnetic fields is an alcohol.

18. Method according to one of the claims 1 to 15 **characterized in that** the substance which influences natural and artificial electric, electrochemical, and electromagnetic fields is cholesterol.

19. Method according to one of the claims 1 to 15 **characterized in that** the substance which influences natural and artificial electric, electrochemical, and electromagnetic fields is a stearin.

20. Method according to one of the claims 1 to 15 **characterized in that** the substance which influences natural and artificial electric, electrochemical, and electromagnetic fields is a protein.

21. Method according to one of the claims 1 to 15 **characterized in that** the substance which influences natural and artificial electric, electrochemical, and electromagnetic fields is a drug.

22. Method according to one of the claims 1 to 15 **characterized in that** the substance which influences natural and artificial electric, electrochemical, and electromagnetic fields is a blood lipid.

23. Method according to one of the claims 1 to 15 **characterized in that** the substance which influences natural and artificial electric, electrochemical, and electromagnetic fields is glucose.

24. Method according to one of the claims 1 to 15 **characterized in that** the substance which influences natural and artificial electric, electrochemical, and electromagnetic fields is blood sugar.

25. Device for the realization of the method according to one of the claims 1 to 24,
wherein:
- electrodes (1) are arranged in relation to a skin surface of a living organism which are operable as sensor and as actuator,
- the electrodes (1) are electrically/electronically connected to an analog switching device (2) which is upstream of an operational amplifier stage (3, 4),
- an analog-digital converter (8) with a digital signal processor (5) is downstream of an impedance preamplifier (3) for conditioning, and
- said digital signal processor is connected to a computer system stage for processing and storing the normalized and digitized measured values.

26. Device according to claim 25, **characterized in that** individual components are arranged separately.

27. Device according to claim 25 or claim 26 **characterized in that** the components are designed in such a way that the data is transmitted wirelessly.

28. Device according to one of the claims 25 to 27 **characterized in that** the components are designed in such a way that the data is transmitted via an optical communication interface.

29. Device according to one of the claims 25 to 28 **characterized in that** the electrodes (1) which are operable as sensor and as actuator are arranged in groups.

30. Device according to claim 29 **characterized in that** the electrodes (1) are arranged in groups in a matrix.

31. Device according to one of the claims 25 to 30 **characterized in that** at least individual components of the system are implanted in the living organism.

32. Device according to one of the claims 25 to 31 **characterized in that** the operational amplifier stage (3; 4) is adapted to the physical properties of the electrodes.

33. Device according to one of the claims 25 to 32 **characterized in that** the analog-digital converter (8) with the digital signal processor (5) is designed scalable according to the number of the applied primary contacts.

34. Device according to one of the claims 25 to 33 **characterized in that** the computer system stage is designed scalable.

35. Device according to one of the claims 25 to 34 **characterized in that** the computer system stage is designed in such a way that it controls the generator (7) for the adaptive stimulation and so the switching between sensor and actuator operation.

36. Device according to one of the claims 25 to 35 **characterized in that** a display (10) is arranged downstream of the computer system stage.

37. Device according to one of the claims 25 to 36 **characterized in that** the computer system stage comprises an integrated storage unit (12).

38. Device according to one of the claims 25 to 37 **characterized in that** the operational amplifier stage (3; 4) comprises an adaptive filter element (4) for the elimination of recognized interferences.

39. Device according to claim 38 **characterized in that** the adaptive filter element (4) is designed as an active multistage type.

40. Device according to one of the claims 25 to 39 **characterized in that** the computer system stage is connected to a depot regulator and controls the same.

41. Device according to claim 40 **characterized in that** the depot regulator is arranged within the living organism.

42. Device according to claim 40 **characterized in that** the depot regulator is arranged outside the living organism.

## Revendications

1. Procédé de détection mobile ou stationnaire de données de fonctionnement et de métabolisme d'un organisme vivant, étant donné que se trouve dans l'organisme vivant une substance influençant des champs électriques, électrochimiques et électromagnétiques naturels et synthétiques, le procédé comprenant les étapes suivantes :
- saisie de signaux électriques qui correspondent à une modification des champs électriques, électrochimiques et électromagnétiques naturels et synthétiques, par des électrodes utilisées alternativement comme capteur et acteur au moyen d'un équipement de commutation (2) analogique monté en amont, électrodes qui sont mises en rapport avec une surface cutanée de l'organisme vivant ;
- enregistrement des signaux saisis dans une mémoire et
- transmission des signaux enregistrés à un équipement de traitement de données.

2. Procédé selon la revendication 1, **caractérisé en ce que** la saisie des signaux électriques se fait continuellement.

3. Procédé selon la revendication 1 ou 2, **caractérisé en ce que** l'influence des électrodes utilisables comme capteur ou comme acteur est globale.

4. Procédé selon l'une quelconque des revendications précédentes, **caractérisé en ce que** les signaux électriques correspondent à des champs électriques, électrochimiques et électromagnétiques naturels et synthétiques qui ont été mesurés au moyen des électrodes en disposition ponctuelle.

5. Procédé selon l'une quelconque des revendications 1 à 3, **caractérisé en ce que** les signaux électriques correspondent à des champs électriques, électrochimiques et électromagnétiques naturels et synthétiques qui ont été mesurés au moyen des électrodes en disposition linéaire.

6. Procédé selon l'une quelconque des revendications 1 à 3, **caractérisé en ce que** les signaux électriques correspondent à des champs électriques, électrochimiques et électromagnétiques naturels et synthétiques qui ont été mesurés au moyen des électrodes en disposition planiforme.

7. Procédé selon l'une quelconque des revendications précédentes, **caractérisé en ce que** les signaux électriques correspondent à une intensité de champ.

8. Procédé selon l'une quelconque des revendications précédentes, **caractérisé en ce que** les signaux électriques correspondent à une densité de lignes de champ.

9. Procédé selon l'une quelconque des revendications précédentes, **caractérisé en ce que** les signaux électriques correspondent à une différence de potentiel.

10. Procédé selon l'une quelconque des revendications précédentes, **caractérisé en ce que** les signaux électriques correspondent à une intensité de courant.

11. Procédé selon l'une quelconque des revendications précédentes, **caractérisé en ce que** les signaux électriques correspondent à une tension électrique.

12. Procédé selon l'une quelconque des revendications précédentes, **caractérisé en ce que** les signaux électriques sont fournis pour représentation graphique.

13. Procédé selon l'une quelconque des revendications précédentes, **caractérisé en ce qu'**une température d'organisme est enregistrée.

14. Procédé selon l'une quelconque des revendications précédentes, **caractérisé en ce qu'**une résistance cutanée est enregistrée.

15. Procédé selon l'une quelconque des revendications précédentes, **caractérisé en ce qu'**un échange de gaz à travers la peau par unité de temps est enregistré.

16. Procédé selon l'une quelconque des revendications précédentes, **caractérisé en ce que** la substance qui influence les champs électriques, électrochimiques et électromagnétiques naturels et synthétiques est le lactate.

17. Procédé selon l'une quelconque des revendications 1 à 15, **caractérisé en ce que** la substance qui influence les champs électriques, électrochimiques et électromagnétiques naturels et synthétiques est un alcool.

18. Procédé selon l'une quelconque des revendications 1 à 15, **caractérisé en ce que** la substance qui influence les champs électriques, électrochimiques et électromagnétiques naturels et synthétiques est le cholestérol.

19. Procédé selon l'une quelconque des revendications 1 à 15, **caractérisé en ce que** la substance qui influence les champs électriques, électrochimiques et électromagnétiques naturels et synthétiques est une stéarine.

20. Procédé selon l'une quelconque des revendications 1 à 15, **caractérisé en ce que** la substance qui influence les champs électriques, électrochimiques et électromagnétiques naturels et synthétiques est une protéine.

21. Procédé selon l'une quelconque des revendications 1 à 15, **caractérisé en ce que** la substance qui influence les champs électriques, électrochimiques et électromagnétiques naturels et synthétiques est une drogue.

22. Procédé selon l'une quelconque des revendications 1 à 15, **caractérisé en ce que** la substance qui influence les champs électriques, électrochimiques et électromagnétiques naturels et synthétiques est un lipide sanguin.

23. Procédé selon l'une quelconque des revendications 1 à 15, **caractérisé en ce que** la substance qui influence les champs électriques, électrochimiques et électromagnétiques naturels et synthétiques est le glucose.

24. Procédé selon l'une quelconque des revendications 1 à 15, **caractérisé en ce que** la substance qui influence les champs électriques, électrochimiques et électromagnétiques naturels et synthétiques est la glycémie.

25. Équipement pour la réalisation du procédé selon l'une quelconque des revendications 1 à 24, étant donné que :
- des électrodes (1) utilisables comme capteur ou comme acteur sont disposés par rapport à une surface de la peau d'un organisme vivant,
- les électrodes (1) sont reliées électriquement / électroniquement à un équipement de commutation analogique (2) qui est monté en amont d'un étage d'amplificateurs opérationnels (3 ; 4),
- un convertisseur analogique-numérique (8) avec processeur de signaux numérique (5) est monté en aval d'un préamplificateur d'impédance (3) qui sert au conditionnement et
- ledit convertisseur est interconnecté avec un étage calculateur qui transforme et enregistre les valeurs de mesure normalisées et numérisées.

26. Équipement selon la revendication 25, **caractérisé en ce que** des unités modulaires individuelles sont disposées séparément dans l'espace.

27. Équipement selon l'une quelconque des revendications 25 ou 26, **caractérisé en ce que** les unités modulaires sont formées de manière à transmettre les données sans fil.

28. Équipement selon l'une quelconque des revendications 25 ou 27, **caractérisé en ce que** les unités modulaires sont formées de manière à transmettre les données par l'intermédiaire d'une interface de communication optique.

29. Équipement selon l'une quelconque des revendications 25 à 28, **caractérisé en ce que** les électrodes (1) utilisables comme capteur et comme acteur sont disposées en groupes.

30. Équipement selon la revendication 29, **caractérisé en ce que** les électrodes (1) sont disposées en groupe dans une matrice.

31. Équipement selon l'une quelconque des revendications 25 ou 30, **caractérisé en ce que** au moins des unités modulaires individuelles du système sont implantées dans l'organisme vivant.

32. Équipement selon l'une quelconque des revendications 25 à 31, **caractérisé en ce que** l'étage d'amplificateurs opérationnels (3 ; 4) est adapté aux propriétés physiques des électrodes.

33. Équipement selon l'une quelconque des revendications 25 à 32, **caractérisé en ce que** le convertisseur analogique-numérique (8) avec processeur de signaux numérique (5) peut être dimensionné en fonction du nombre de contacts primaires utilisés.

34. Équipement selon l'une quelconque des revendications 25 à 33, **caractérisé en ce que** l'étage calculateur peut être dimensionné.

35. Équipement selon l'une quelconque des revendications 25 à 34, **caractérisé en ce que** l'étage calculateur est formé de manière à commander la génératrice (7) qui sert à la stimulation adaptative et, ainsi, la commutation entre le fonctionnement comme capteur et comme acteur.

36. Équipement selon l'une quelconque des revendications 25 à 35, **caractérisé en ce qu'**un visuel (10) est monté en aval de l'étage calculateur.

37. Équipement selon l'une quelconque des revendications 25 à 36, **caractérisé en ce que** l'étage calculateur présente une unité de mémoire (12) intégrée.

38. Équipement selon l'une quelconque des revendications 25 à 37, **caractérisé en ce que** l'étage d'amplificateurs opérationnels (3 ; 4) présente un élément filtrant adaptatif (4) pour l'élimination des signaux perturbateurs détectés.

39. Équipement selon la revendication 38, **caractérisé en ce que** l'élément filtrant adaptatif (4) est réalisé de manière à concevoir activement sur plusieurs étages.

40. Équipement selon l'une quelconque des revendications 25 à 39, **caractérisé en ce que** l'étage calculateur est relié comme commande à un régulateur de dépôt.

41. Équipement selon la revendication 40, **caractérisé en ce que** le régulateur de dépôt est disposé au sein de l'organisme vivant.

42. Équipement selon la revendication 40, **caractérisé en ce que** le régulateur de dépôt est disposé hors de l'organisme vivant.
